# EUROPEAN PATENT SPECIFICATION

(11) **EP 3 301 448 B1**
(45) Date of publication and mention of the grant of the patent: **03.03.2021**
(21) Application number: 17197902.4
(22) Date of filing: 25.10.2013
(51) Int. Cl.: G01N 33/574

(54) **METHYLGLYOXAL AS A MARKER OF CANCER**
METHYLGLYOXAL ALS KREBSMARKER
METHYLGLYOXAL COMME MARQUEUR DU CANCER

(30) Priority: 25.10.2012 WO PCT/EP2012/071163
(43) Date of publication of application: 04.04.2018
(62) Divisional of application: 13783073.3
(73) Proprietor: Association pour la Recherche Thérapeutique Anti-Cancéreuse, 75015 Paris (FR)
(72) Inventor: BELPOMME, Dominique, 75014 PARIS (FR); IRIGARAY, Philippe, 54410 LANEUVEVILLE DEVANT NANCY (FR)
(74) Representative: Bourgouin, André

(56) References cited:
- US-A1- 2009 104 596
- BISWAS: "Elevation of serum methylglyoxal may be used as a screening marker in oral premalignant lesions", BIOMEDICAL RESEARCH, vol. 22, no. 3, 1 January 2011 (2011-01-01), page 273, XP055065248, ISSN: 0388-6107
- TOSHIKI MATSUURA ET AL: "Studies on methylglyoxal. II. Changes of methylglyoxal level accompanying the changes of glyoxalase I and II activities in mice bearing L1210 leukemia and Sarcoma 180.", CHEMICAL & PHARMACEUTICAL BULLETIN, vol. 34, no. 7, 1 January 1986 (1986-01-01), pages 2926-2930, XP055065258, ISSN: 0009-2363, DOI: 10.1248/cpb.34.2926
- ANTOGNELLI CINZIA ET AL: "Overexpression of glyoxalase system enzymes in human kidney tumor", CANCER JOURNAL, vol. 12, no. 3, May 2006 (2006-05), pages 222-228, XP008162759, ISSN: 1528-9117
- TOMOKO OYA-ITO ET AL: "Heat-shock protein 27 (Hsp27) as a target of methylglyoxal in gastrointestinal cancer", BIOCHIMICA ET BIOPHYSICA ACTA. MOLECULAR BASIS OF DISEASE, AMSTERDAM, NL, vol. 1812, no. 7, 23 March 2011 (2011-03-23), pages 769-781, XP028205363, ISSN: 0925-4439, DOI: 10.1016/J.BBADIS.2011.03.017 [retrieved on 2011-04-06]
- SYNOLD TIMOTHY ET AL: "Advanced Glycation End Products of DNA: Quantification of N-2-(1-Carboxyethyl)-2 '-deoxyguanosine in Biological Samples by Liquid Chromatography Electrospray Ionization Tandem Mass Spectrometry", CHEMICAL RESEARCH IN TOXICOLOGY, vol. 21, no. 11, November 2008 (2008-11), pages 2148-2155, XP008162758, ISSN: 0893-228X
- NYANDIEKA ET AL: "Searches for clinically exploitable biochemical differences between normal and cancer cells: Glyoxalase system in malignancy and its relation to cellular proliferation", CLINICAL BIOCHEMISTRY, ELSEVIER INC, US, CA, vol. 11, no. 4, 1 January 1978 (1978-01-01), pages 150-155, XP023600288, ISSN: 0009-9120, DOI: 10.1016/S0009-9120(78)90349-1 [retrieved on 1978-01-01]
- ANTOGNELLI C [0000-0002-8259-680X] ET AL: "Methylglyoxal acts as a Tumor-Promoting factor in Anaplastic Thyroid Cancer", CELLS 20190601 MDPI AG CHE, vol. 8, no. 6, 1 June 2019 (2019-06-01), ISSN: 2073-4409

## Description

The present disclosure discloses a new, reliable, sensitive and easy to handle diagnostic and prognostic test for cancer in human or animal subjects. The present Inventors have shown here for the first time that increased levels of methylglyoxal (MG) in biological samples of cancer-bearing subjects are highly positively correlated with the development and progression of cancer cells that are metabolically active. This highlights that cancer cells produce and release significantly higher amounts of MG than normal cells in the tumor as well as in extracellular fluids in the organism, and so it is possible to obtain a reliable and sensitive diagnosis and prognosis test of cancer from a unique blood sample. The present invention therefore relates to an *in vitro* method for monitoring in cancer-bearing subjects; by measuring the presence of MG.

### BACKGROUND OF THE INVENTION

With the growing number of cancer cases that are being diagnosed worldwide and the persisting high number of deaths due to late discovery of the disease, the identification of new biomarkers for both early detection and targeted therapeutic interventions is widely accepted to be crucial, both for cancer prevention and for better outcome in treated cancer patients. Cancer is the second-highest cause of death worldwide, with lung, colon, breast (female), pancreas and prostate (males) cancers being most common.

Currently, the most used cancer diagnostic and prognostic indicators are based on the morphological and histological characteristics of tumors, as there are no available blood biomarkers with sufficient sensitivity and specificity for diagnosis; and only a few biomarkers exist for therapeutic monitoring and the prognosis evaluation of cancer.

The US Food and Drug Administration (FDA) has defined a biomarker as a molecular characteristic that is objectively measured and evaluated and which indicates normal biologic processes, pathogenic processes or pharmacologic responses to a therapeutic intervention. Such biomarkers could be produced either by the tumor itself or by the body in response to the malignant pressure towards normal cells turning them becoming cancerous. According to the US National Cancer Institute (NCI), biomarkers could be used for cancer screening, risk assessment, early diagnosis of disease, monitoring, prognosis evaluation, therapeutic decision making and prediction of response to therapy.

However, a major challenge in harnessing this potential of oncology biomarkers is that cancer initiation and promotion and tumor progression are complex processes involving various abnormal genetic and epigenetic molecular events and cellular interactions.

Malignant transformation leads to specific and non-specific phenotypic cell signature changes, hence to the clonal selection and progression of cancer cells in the organism. In addition cancer may result from exposure to multiple and diverse environmental carcinogenic agents, such as chemicals, radiation and/or microorganisms; especially in genetically susceptible hosts (Belpomme et al, Environ Res 2007; Irigaray and Belpomme, Carcinogenesis 2010).

As a consequence of such complexity of the carcinogenic processes, tumors vary widely in etiology and pathogenesis, so cancer consists of more than 200 distinct diseases affecting over 60 human organs. This complexity is also why, although many bioassays have looked for correlations between clinical oncologic endpoints and biological markers, there are still very few clinically useful biomarkers to aid oncologists in decision-making and patient care, and-critically-no available single biomarker that can detect all or even many types of cancer. The present invention reconciles many of these limitations, by providing a new single biomarker that allows the detection of many types of cancer, through a simple measurement in biological samples of the patient / subject. This measurement is very reproducible; so as to detect cancer, even at early stage.

BISWAS et al., BIOMEDICAL RESEARCH, vol. 22, no3, 1 January 2011 page 273, disclose that serum MG level was found to be significantly elevated in oral premalignant lesions and significantly decreased in established malignant disease patients.

TOSHIKI MATSUURA et al., CHEMICAL & PHARMACEUTICAL BULLETIN vol. 34, n°.7, 1 January 1986, pages 2926-2930, disclose that, in mice, the initial proliferation of the tumor cells is accompanied by a decrease of MG and further development of the tumor cells results in an increase of MG level.

ANTOGNELLI CINZA et al., CANCER JOURNAL vol. 12, n°.3, pages 222-228, disclose a significant decrease in MG concentrations in the cancerous tissues.

Basically, the present invention consists in measuring the production level of the metabolic byproduct methylglyoxal (MG), by detecting and quantifying the amount of this molecule in a biological sample from a subject.

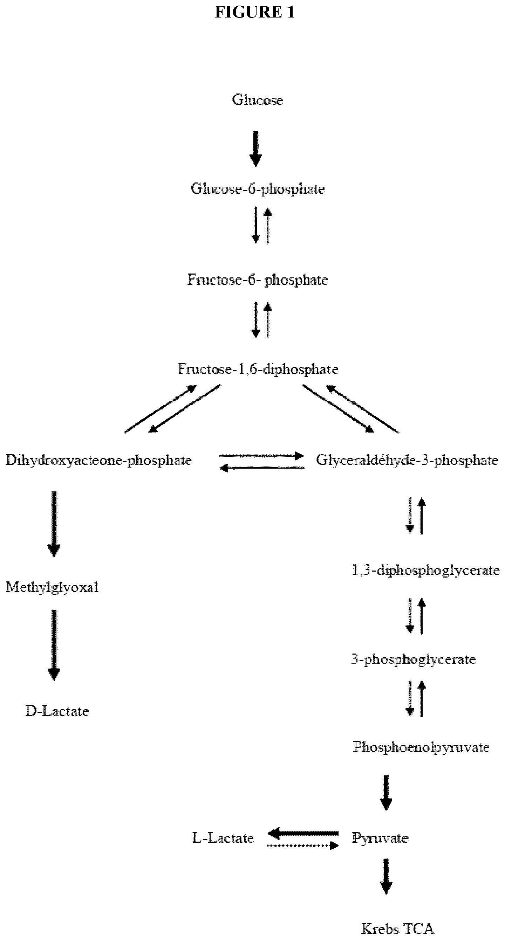

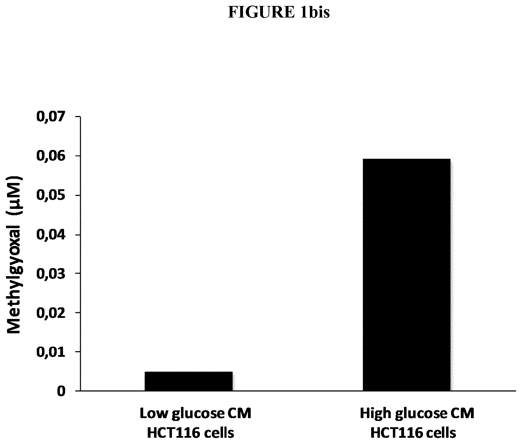

*Figure 1* is a schematic diagram showing the glycolysis metabolic process and the methylglyoxal (MG) formation in eukaryotic cells.

The whole glycolytic pathway for energy is anaerobic (no oxygen used). Normal cells in aerobic conditions enter the Krebs tricarboxylic acid (TCA) cycle to produce adenosine triphosphate (ATP); whereas still in aerobic conditions, the Warburg effect leads many cancer cells, instead of entering the Krebs TCA, to increase glycolysis (i.e. "aerobic glycolysis"). During the glycolysis process, the MG pathway bypasses the classical glycolytic Embden-Meyerhof-Pamas pathway and is a metabolic *cul-de-sac*; consequently this pathway leads to the formation of MG and D-lactate as waste end/by-products while the glycolytic Embden-Meyerhof-Pamas pathway leads either to the formation of pyruvate then to the Krebs TCA in aerobic conditions, or to the formation of L-lactate from pyruvate in anaerobic conditions. Any deficiency in the Krebs TCA, or in the respiratory chain, as it is the case in many cancer cells, increases glycolysis for compensating ATP production and consequently MG formation via an increased formation of dihydroxyacetone-phosphate.

*Figure 1* *bis* shows the production of MG in the culture medium of a human carcinoma cell line (HCT16) depending on the presence of either a low or a high glucose concentration in the culture medium. MG quantification in the conditioned medium (CM) was done by using Liquid chromatography-tandem mass spectrometry (LC-MS/MS) analysis. In high glucose condition, cancer cells produce 10 fold higher MG than in low glucose condition a finding suggesting that MG synthesis and release by cancer cells directly depend on glucose consumption and metabolism.

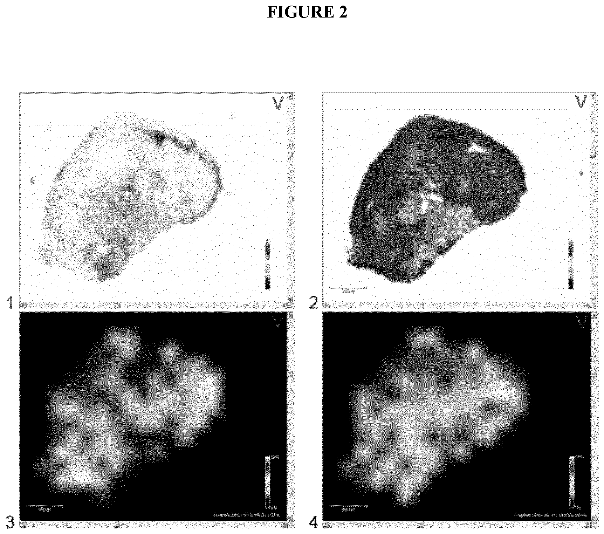

*Figure 2* shows the MG production in a malignant PRO cell clone by using direct tissue analysis by MALDI-TOF/TOF mass spectrometry. PRO cell clones were initially obtained from a colon adenocarcinoma induced by 1,2-dimethylhydrazine administration. The tumor is represented without coloration and after coloration with hematoxilin-Eosin-Safran (HES) in scans 1 and 2 respectively. MG intra-tumoral detection by MALDI-TOF/TOF mass spectrometry, is represented in scans 3 and 4 obtained respectively from 91 Da and 118 Da 2MQX molecular fragment analysis. Note that in the necrotic zone that predominates in the middle and lower part of the tumor in 1 and 2, MG in 3 and 4 appears to be less detected, while it appears to be mostly detected in some zones highly coloroured by HES.

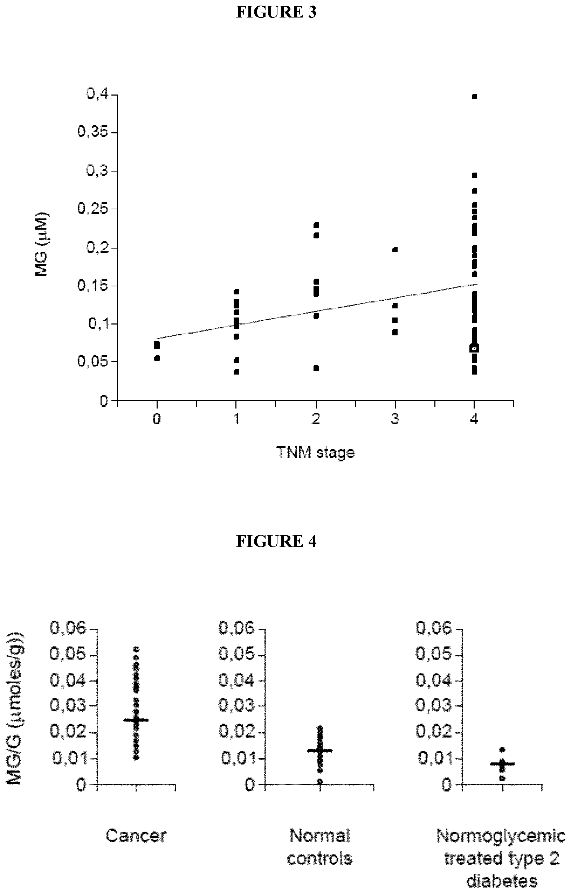

*Figure 3* shows the significant positive correlation between MG blood levels and tumor stages in patients with cancer. Whatever the stage of the cancer, most MG blood level values are above the normal (non-cancerous) control value of 0.06 µM of MG (p=0.0109), showing that systematic measurement of MG in the blood is an efficient tool for diagnosing cancer; critically enabling easy and early detection and screening.

*Figure 4* shows the difference (p <0.01) of the ratio of blood MG to blood Glucose (MG/G index) between cancer patients and controls (either normal subjects or normo-glycemic (treated) type 2 diabetes patients). Note that there is no significant difference for MG blood levels between normal controls and subjects with normo-glycemic treated type 2 diabetes.

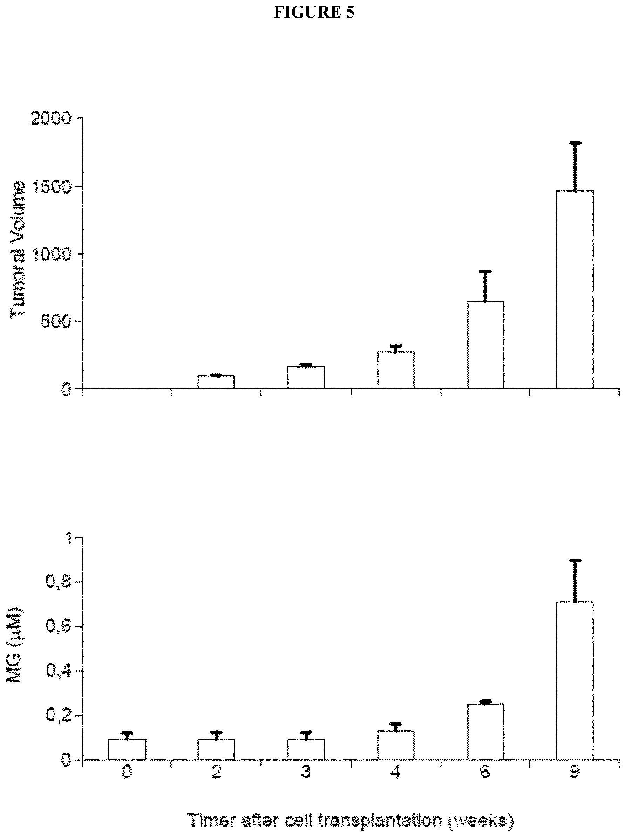

*Figure 5* discloses the significant positive correlation between MG blood levels and tumoral volume in BD-IX rats developed after transplantation of PRO tumorigenic cancer colonic cells (p<0.001). The PRO and REG cell clones were initially derived from a single colon adenocarcinoma induced by 1,2-dimethylhydrazine in BD- IX rat.

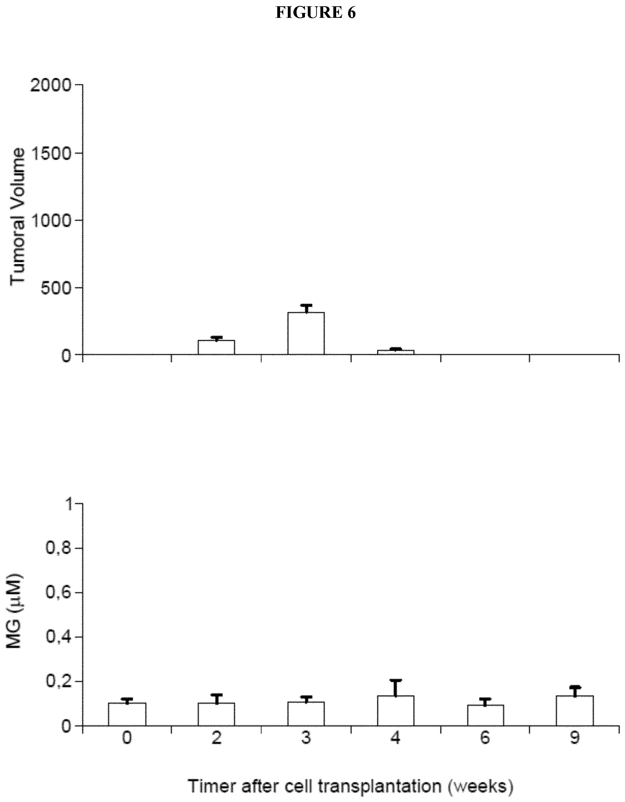

*Figure 6* shows that when injected sub-cutaneously into syngenic host, PRO cells like parental cells induce progressive tumors, whereas REG cells induce tumors that regress after 3 weeks. The tumoral graft of PRO cells is associated with a constant progression of the tumor volume, so note the positive correlation between MG blood levels and tumoral volume; three weeks after transplantation there is a constant progression of MG blood levels. The tumoral graft of REG cancer cells is rejected 3 weeks after transplantation and MG in the blood remains at a low level during the whole experimental period. By comparing the results obtained with the PRO tumorigenic cancer cell clone (Fig 5), this suggests that actively progressing cancer and more particularly proliferative tumorigenic cancer cells synthetize large amounts of MG; while non-progressing cancer, more particularly non-proliferative non-tumorigenic cancer cells, cannot.

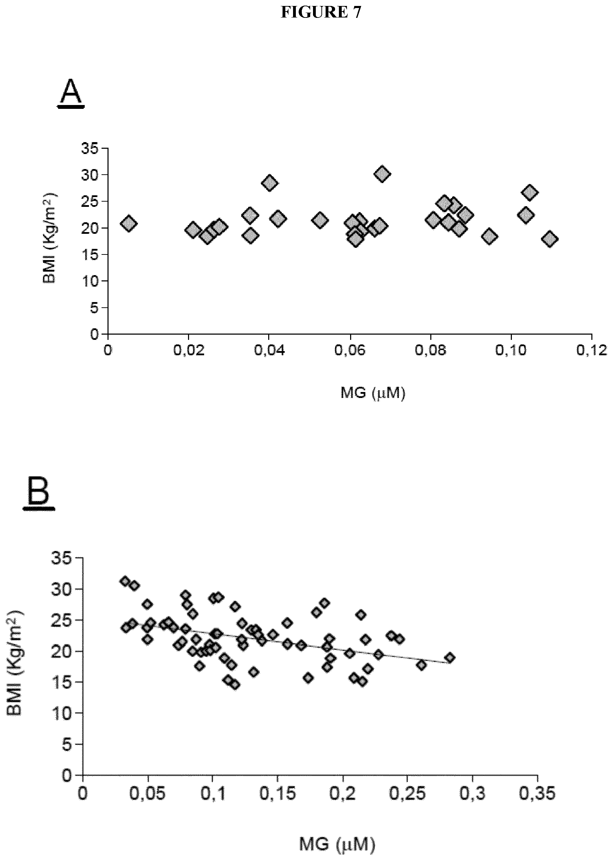

*Figure 7* shows that in cancer patients (B), MG blood levels are significantly inversely correlated with body mass index (BMI) (p=0.0064); whereas there is no correlation of MG with BMI in normal subjects (A). This indicates that MG is produced by cancer cells.

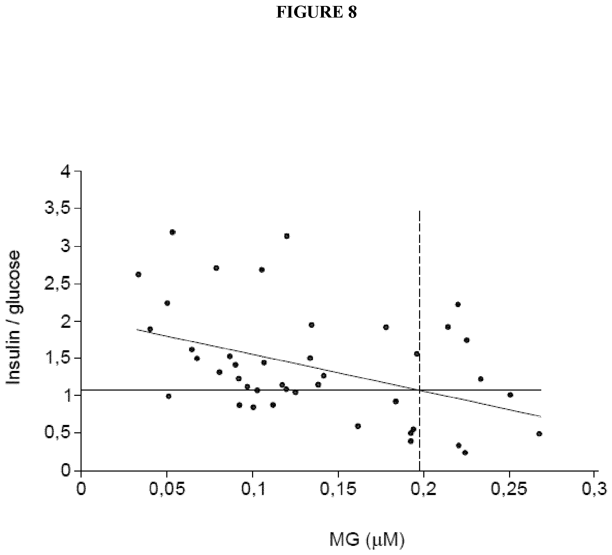

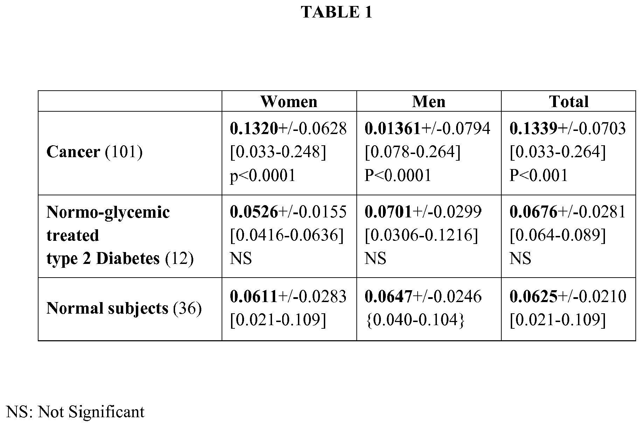

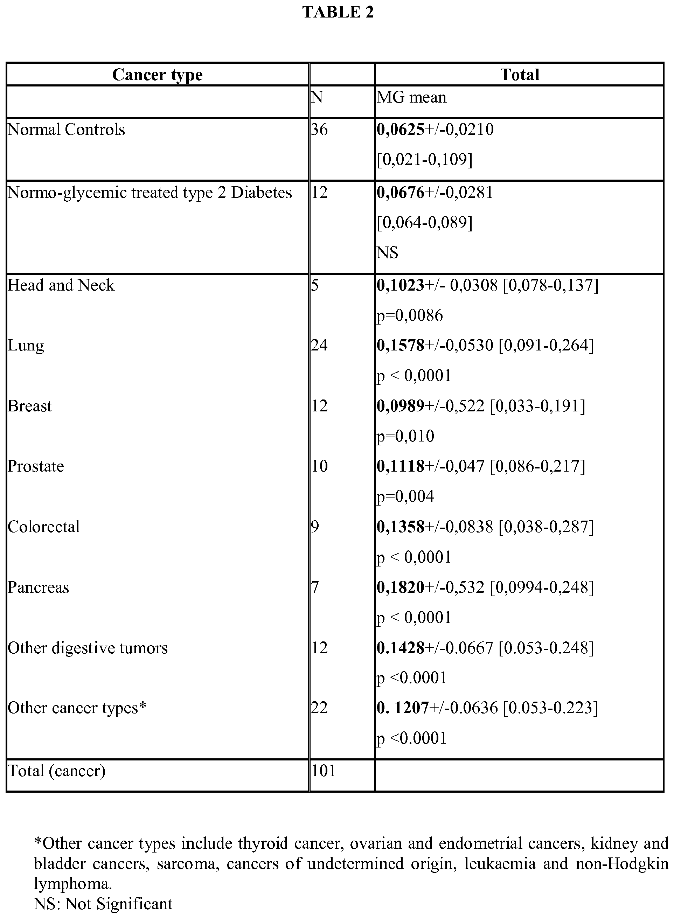

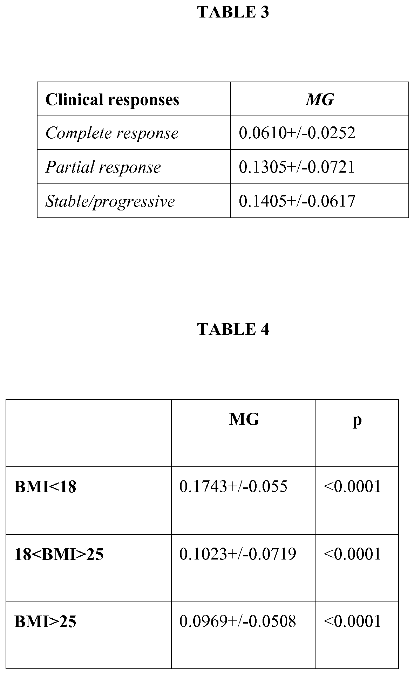

*Figure 8* shows that in cancer patients there is a significantly inverse relationship between insulin/glucose ratio (I/G) index and MG blood levels, whereas the I/G index in normal healthy subjects remains constant. Consequently the intersection of the 2 curves allows the individualization of a critical point at which 0.2 µM blood MG level is referred as the "cachexia-associated control value" above which, insulin resistance is higher and insulin secretion lower than in normal subjects; such that cancer patient are entering cachexia or severe pre-cachexia.

*Table 1* shows MG blood level mean values (± standard errors and confidence intervals) (in µM) in cancer patients, in comparison with normal subjects and patients with normo-glycemic treated type 2 diabetes used as controls.

*Table 2* shows MG blood level mean values (± standard errors and confidence intervals) (in µM) in cancer patients according to tumor types in comparison with normal subjects and normo-glycemic treated type 2 diabetes patients used as control.

*Table 3* discloses the mean values (± standard errors) of MG blood levels (in µM) in treated cancer patients according to clinical responses; i.e. complete response, partial response or stable / progressive disease, as determined by direct clinical tumor and/or tumor measurement by using imaging techniques. In two patients with apparently complete clinical response as determined by classical imaging techniques, MG blood levels were above normal values. In these two patients, cancer relapsed 3 and 7 month later.

*Table 4* discloses the mean (± standard errors) of MG blood levels (µM) in different cancer patients according to their BMI. The distinction between the three BMI categories is highly statistically significant and pre-cachectic or cachectic states (BMI< 18) are associated with significant higher MG blood levels in comparison with cancer patients with overweight / obesity (BMI>25).

Biological samples: As used herein, the term "biological samples" refers to a variety of sample types obtained from patients or from normal individuals, for their use in a diagnostic monitoring assay. Said biological samples encompass any extracellular fluids such as blood, serum, plasma.

Methylglyoxal: In the present invention, "Methylglyoxal" refers to the discovery that cancer cells produce and release significant higher amounts of methylglyoxal (MG) than normal cells do, so that MG can be measured and quantified in tumor and extracellular fluids in an organism, particularly in the peripheral blood after MG is released from cancer cells. MG is evidenced in samples by measuring *in vitro* the fraction of free MG that exists spontaneously in the organism; or by measuring the total amount of free MG that corresponds to the free MG that exists spontaneously in samples in addition to the MG that can be recovered from the reversibly ligand-bound MG after the samples have been treated by use of a technique similar or identical to that of Chaplen (Chaplen et al, Anal Biochem 1996; Chaplen et al., PNAS 1998). Such methods have been initially developed to measure intracellular reversibly ligand-bound MG.

Consequently, the MG whose level is measured by the method of the invention corresponds to the level of free MG molecules measured in the tumor or in the body fluids of individuals, more particularly in the peripheral blood because that makes clinical use of the biomarker very simple. However, as previously indicated, the method of the invention does not rely exclusively on the measurement of the free MG that is present spontaneously in a tumor or in the extracellular compartment in the organism, but it relies also on the measurement of the free MG that is recovered after *in vitro* treatment of the reversibly ligand-bound MG. However when the terms "MG", "MG production" or "MG production levels" are used herein without further definition, the MG level corresponds to the free molecules that are present spontaneously in the sample considered, and not to the total amount of free MG that can be recovered from the sample, whose measurement is however also included in the invention.

The term "biological samples" includes samples that have been manipulated in any way after their procurement.

The said biological sample can be "treated" prior to MG analysis, such as by: preparing plasma from blood, eliminating cells from the sample or making enrichment of cell population, diluting viscous fluids, or the like. Methods of treatment can involve filtration, distillation, concentration, inactivation of interfering compounds, cell lysis; for example by sonication, addition of reagents, cell fixation or solid tissue fixation prior to MG analysis. Examples of so called treated samples prior to MG analysis use techniques for recovering intracellular and/or extracellular reversibly ligand bound MG (Chaplen et al, Anal Biochem 1996; Chaplen et al, PNAS 1998).

Subjects, individuals, patients: The terms "subjects" or "individuals" used herein refers to persons (or animals) of any age or gender, whether healthy or suffering from disease; while the term "patients" refers to disease-bearing subjects or individuals, such as cancer or diabetes-bearing ones.

The terms 'healthy subject/s' and 'healthy individual/s' refer to non-symptomatic subjects or individuals that have been proved to be without any detectable disease by using usual medical tests. More precisely, these terms refer to people that have been proved to be free from cancer, diabetes, chronic uremia, arterial hypertension and Alzheimer disease.

Cancer or leukemia: These terms refer to tumors whose cells exhibit an aberrant malignant phenotype characterized by several recognized and validated hallmarks which mainly include autonomous growth in the organism and loss of cell proliferation regulation. These hallmarks have been more precisely reviewed and analyzed recently (Hanahan and Weinberg, Cell 2011). By contrast the term "tumor" refers to cells that can exhibit a malignant or non-malignant phenotype. The term "benign tumor" is used to characterize tumors whose proliferative capacity remains limited because cells do not harbor a malignant phenotype.

There is no particular limitation regarding which cancer types be identified by the method of the present invention: they include solid and non-solid cancers, which encompass both epithelial or non-epithelial types.

Cancers of epithelial origin include all histological types such as adenocarcinoma and squamous cell carcinoma; and all localizations for example cancers of the head and neck (i.e. oral cavity, lingual, oropharynx, pharyngeal, laryngeal, etc.), bronchus & lung, breast, gastric, colorectum, pancreatic, hepatic (and all other digestive types), cervix and endometrial uterus, ovarian, urogenital (prostate, bladder, renal); etc. Non-epithelial cancers consist in particular of any type of leukemia, lymphoma, melanoma or sarcoma.

Other cancers also can be identified by the present invention, for example, testicular cancer, dysgerminoma, glioblastoma, astrocytoma, mesothelioma, Ewing sarcoma, childhood cancers and HIV-related tumors, among others.

By "early detection" of cancer, it is herein understood to mean the detection or identification of an established sub-clinical (not obviously diagnosable) microscopic already metabolically active cancer in non-symptomatic subjects.

By "screening" of cancer it is understood to mean the systematic detection of metabolically active cancer or precancerous lesions in a population of non-symptomatic individuals.

By "diagnosis" of cancer it is understood to mean the detection of an already macroscopic progressive cancer in symptomatic patients. The detecting/diagnosing method of the invention is thus not dedicated to detect so-called precancerous lesions that may be evidenced in tissue biopsies but which may not necessarily progress into true metabolically active microscopic cancer. Rather, this invention easily and reliably detects truly proliferative and progressing cancer. This may occur in non-symptomatic subjects in the form of sub-clinical microscopic lesions, or in symptomatic subjects in the form of more advanced lesions, before it can be evidenced by the usual available clinical diagnostic tools. Since MG levels relate to the metabolic activity of progressing cancer cells, the detecting/diagnosis method of the present invention can be used not only for screening of metabolically active cancer in non-symptomatic subjects, but also for the progression of proliferative cancer in symptomatic subjects and therefore for the estimation in such subjects of the likelihood that the cancer will progress clinically; before cancer progression will be evidenced by usual available clinical tools.

As used herein, the terms "MG normal control values" or "MG reference values" refer to specific value and/or value intervals that has been determined from normal disease-free subjects, particularly cancer and diabetes-free (i.e., healthy donors). The normal control value of 0.6 µM ± 0.02 used herein is the mean value of MG production level in whole blood samples from healthy donors, measured by High-performance liquid chromatography (HPLC) according to a method described below. Thus, in a preferred embodiment, said normal control value is the MG production level which has been measured in a biological sample - preferably a blood sample - from subjects who do not suffer from cancer or diabetes, and who are also otherwise disease free. The reference may be a single overall value, such as a median or mean value, or it may be different values for specific subpopulations of subjects. A person skilled in the art will appreciate that the ratio between the MG production level in the test sample and the MG control value can depend on what type of control value is used.

The method of the invention enables medical and biomedical professionals to determine if a non-diabetic subject has a high or low risk of having a cancer. This cancer probability is estimated to be proportional to the MG production level in the tested subjects for values above the normal control value.

A non-diabetic subject is said to have a "high risk of having a cancer", when the MG production level in said biological sample is higher than the said normal control value: that means the subject has a high risk of having a cancer at the time of the collection of the biological sample albeit the cancer may or may not be detectable yet by usual available diagnostic tools. In other words, the subject is considered to have a higher probability to have a cancer as compared to the normal population when the MG production level in the tested subject is above the MG normal control value. More precisely in the context of the invention, a subject is said to have "a high risk of having a cancer" when he/she has a likelihood higher than 50%, preferably 70%, better 90%, ideally 95%, of having a cancer.

In contrast, the risk of having a cancer is low when the MG production level in the biological sample of the tested subject is within the normal control value interval and a fortiori when the MG production level is below the inferior limit of the normal control value interval. This means that the subject has a low probability to have a cancer or is not developing a cancer at the time of the collection of the biological sample.

In the context of the invention, the subject has a low risk of having a cancer when he/she has a probability of having a cancer lower than 10%, preferably lower than 5%, as compared with the normal population. In other words, the subject has a 90%, preferably 95% probability to be cancer-free. In the context of the present invention, the MG production level in a subject's sample is said to be "significantly higher" or "higher" than the control value, when said MG level is 1.5 fold higher, more reliably 2 fold, most reliably 3 fold higher than said control value. The subject is said to have a high risk of having a cancer (typically between 50% - 80% risk), when its MG production level is 2 fold higher than said control value. An even higher cancer risk (typically between 80% - 100% risk) is when its MG production level is 3 fold higher than said control value. In contrast, the MG production level of a tested subject is said to be "significantly lower" or "lower" than the control value, when said MG production level is 1.5 fold lower, preferably 2 fold, and more preferably 3 fold lower than said control value. Conversely, the subject is said to have a low risk of having a cancer (typically between 20% - 50% risk), when its MG production level is 2 fold lower than said control value, and an even lower risk (typically between 0 - 20% risk) when its MG production level is 3 fold lower than said control value. Finally, the MG production level of a tested subject is said to be "similar to a control value" if the ratio between said MG production level and said MG control value is between 0.8 and 1.2, preferably between 0.9 and 1.1, more preferably between 0.95 and 1.05.

Glucose is a monosaccharide with formula C₆H₁₂O6 or H-(C=O)-(CHOH)₅-H whose five hydroxyl (OH) groups are specifically arranged on its six-carbon backbone, normally as a ring. In its fleeting chain form, the glucose molecule has an open (as opposed to cyclic) and unbranched backbone of six carbon atoms, C-1 through C-6; where C-1 is part of an aldehyde group H(C=O)-, and each of the other five carbons bears one hydroxyl group -OH (the remaining bonds of the backbone carbons are saturated with hydrogen atoms -H).

In water-based solutions, the open-chain form of glucose (either 'D-' or 'L-' handed) exists in equilibrium with several cyclic isomers to glucose, each containing a ring of carbons closed by one oxygen atom. In aqueous solution, over 99% of glucose exists as pyranose. The open-chain form is limited to about 0.25% and furanose is in negligible amounts. The terms "glucose" and "D-glucose" are generally used for these cyclic forms as well. The ring arises from the open-chain form by a nucleophilic addition reaction between the aldehyde group - (C=O)H at C-1 and the hydroxyl group -OH at C-4 or C-5, yielding a group -C(OH)H-O-. The open isomer D-glucose gives rise to four distinct cyclic isomers: α-D-glucopyranose, β-D-glucopyranose, α-D- 25 glucofuranose, and β-D-glucofuranose; which are all chiral.

The other open-chain isomer L-glucose similarly gives rise to four distinct cyclic forms of L-glucose.

In the context of the present invention, the term "glucose" designates any of the glucose isomers, either cyclic or in open-chain form.

Once the levels of MG and glucose have been determined in the tested biological sample, it is possible to calculate the so called MG/G index, which is the ratio between the level of MG and the level of glucose in the tested biological sample. This ratio, expressed in µmoles/g is then compared to a normal control ratio to determine if the patient is suffering from cancer.

Because cancer cells consume higher amount of glucose and produce and release higher amount of MG than normal in the context of the present invention, the metabolic activity of cancer cells in the extracellular compartment in the organism is characterized by an MG/G index, defined as the ratio of the blood MG production level expressed in nmoles/g on the blood glucose level (G) expressed in mmoles/l according to the formula: MG/G index = MG/G in which MG/G is expressed in µmoles/g.

In non-cancerous diabetic patients there is a positive correlation between the glucose level, the glycated hemoglobin HbAlc percentage and the MG production level in the blood (Beisswenger et al, Diabetes 1999). In other words, in such patients, the higher the glycemia, the higher is the glycated hemoglobin HbAlc percentage and the higher is the circulating MG blood level. This explains why in the blood of non-cancerous diabetic patients there is a simultaneous increase in the levels of both glucose and MG. In contrast, in cancer-bearing diabetic patients, a significant increase in the MG/G index relates to the fact that, because of their higher glucose consumption (the so-called "glucose pump" effect) and their higher glycolytic activity (Hsu and Sabatini, Cell 2008; Koppenol et al., Nat Rev Cancer 2011), cancer cells produce and release significantly higher amounts of MG in the blood, as the Inventors have shown herein (see below); while due to their specific glucose pump effect, they simultaneously tend to decrease the extracellular glucose in the organism; explaining why, at the difference of what occurs in diabetic patients, glycemia remains normal in cancer patients, even an advanced state.

In a preferred embodiment, the control ratio (hereafter referred as "normal MG/G control index") is the MG/G ratio index which has been determined from biological samples - preferably blood samples of subjects who do not have cancer nor diabetes, preferably of healthy subjects.

In the context of the invention, the normal MG/G control ratio index is about 0.01, which corresponds to the intermediate between the median MG/G index value obtained from the blood of healthy donors and the median MG/G index value obtained from the blood of non cancerous normo-glycemic treated diabetic subjects (see Fig 4).

In the context of the present invention, the MG/G index of a diabetic patient is "significantly higher" or "higher" than the normal MG/G control index, when said MG/G index is 1,5 fold higher, preferably 2 fold and more reliably 3 fold higher than said normal MG/G control index. The diabetic patient is said to have a high risk of having a cancer (typically between 50% - 80% risk) when his / her MG/G index is 2 fold higher than said control index, and an even higher risk (typically between 80 - 100% risk) when his / her MG/G index is 3 fold higher than said control index.

In contrast, the MG/G index of a diabetic patient is said to be "significantly lower" or "lower" than the normal MG/G control index, when said MG/G index is 1,5 fold lower, preferably 2 fold, and more preferably 3 fold lower, than said normal MG/G control index. The patient is said to have a low risk of having a cancer (typically between 20% - 50% risk), when its MG/G index is 2 fold lower than said normal MG/G control index, and an even lower risk (typically 0 - 20% risk) when its MG/G index is 3 fold lower than said normal MG/G control index. Finally, the MG/G index of a diabetic patient is said to be "similar to the control index" if the ratio between said MG/G index and said control index is comprised between 0.8 and 1.2, preferably between 0.9 and 1.1, more preferably between 0.95 and 1.05.

As used *herein*, the terms "cancer staging" or "cancer stages" designates the clinical classification of cancer into the four internationally recognized categories called stages I, II, III, and IV. These four prognostic stages are determined at diagnosis time, i.e. before any anticancer treatments have been administered. Staging has been largely based on the 'TNM' classification of cancer (where T = size & tissue invasion; N = involvement of regional lymph nodes, M = distant metastasis). Depending of the tumor type, staging may be determined with other classification systems. So while for example the TNM classification is commonly used for breast cancer, bronchus cancer and head and neck cancers; the FIGO classification (International Federation of Gynecologists and Obstetricians) is commonly used for ovarian carcinoma and a modified Dukes classification for colon cancers. Thus in the context of the present invention, the Inventors categorized cancers into the four stage I, II, III and IV prognostic classification by considering the most commonly used classification for each cancer type. In addition stage 0 was restricted to in situ non invasive cancers.

The terms "treatment", "treating", "treat" and the like used herein generally refer to obtaining an anticancer pharmacologic and/or physiological response. The effect may be prophylactic in term of preventing cancer progression in non-symptomatic subjects, and/or it may be *stricto sensu* therapeutic in symptomatic patients, in order to obtain a partial or complete stabilization or cure of cancer.

More precisely as used herein the term "anticancer treatment" refers either to chemotherapy, radiotherapy, surgery or any recognized biological or chemical therapies used by the practitioners. Existing treatments are summarized for example on the website of the US National Cancer Institute (NCI) at: http://www.cancer.gov/cancertopics/treatment/types-of-treatment.

The growth doubling time of a tumor is defined as the period of time that is necessary for a tumor to double in volume (or more precisely a doubling of the number of non-stromal tumoral cells).

As used herein, the term "tumoral response" refers to the different internationally recognized modalities of tumor evolution after an anti-cancer treatment has been administered to a cancer patient whose disease is perceptible, i.e. wherein the tumoral response can be assessed directly by measuring tumor clinically and/or indirectly by measuring tumor by using available imaging techniques. The type of response is determined after a certain time interval during which the anticancer treatment has been administered. The evaluation consists in comparing the measurements made after treatment to those made before treatment. There are four response categories: (1) progressive tumor: the increase in tumor volume is more than 25%; (2) stable tumor: the increase in tumor volume is less than 25% and the tumor shrinkage is less than 25 %; (3) partial response: the tumor shrinkage is more than 25% but less than 100%; and (4) complete response: the measured tumor volume is null, i.e. the tumor is undetectable by the means of available techniques.

The time interval between the first and second biological samples, i.e. the time at which the second biological sample must be provided to assess prognosis or therapeutic response mainly depends on the growth doubling time of the tumor; the shorter the doubling time is, the shorter the time interval should be. By itself, the growth doubling time depends on tumor type and treatment efficacy. So in the case of rapidly growing tumor the time interval for sampling could be one, two or three months, while in slowly growing tumor it could be four, five, six months or even more.

It is considered herein that a said anti-cancer treatment is not efficient on said patient if, when the second biological sample is provided one, two or three months or even six months after the first biological sample, depending on the doubling time of the tumor, the MG production level is 2 fold and more preferably 3 fold higher than said MG production level in the first sample. In contrast, it is said that the said anti-cancer treatment is efficient on said patient if; when the second sample is obtained for example one, two, three months or even six months after the first sample, depending on the growth doubling time of the tumor; the second MG production level is 2 fold and more preferably 3 fold lower than the MG production level in the first sample.

Survival depends on tumor type, stage and treatment. By "long- term survival", it is understood herein that the said tested subjects will have a survival of at least 12 months, preferably 3 years and more preferably 5 years after the sample collection has been performed. On the other hand, by "short-term survival", it is understood herein that the said tested subjects will live no more than 5 years, probably less than 3 years, and more probably less than 12 months after the sample collection has been performed.

In the context of the present disclosure, it is considered that the likelihood of a patient to be cured or even survive a long time is low when the determination of the MG production level in a second sample obtained one month, two months, three months or even six months after a first sample, is 2 fold and more definitely 3 fold higher than said MG production level in the first sample. In contrast, it is considered that the patient has a higher chance of long term survival or even can be definitively cured when the MG production level in a second sample obtained three months, preferably six months and more preferably one year after the first sample is 2 fold, more preferably 3 fold lower than the MG production level in a first sample and ideally when the MG production levels measured in several samples after the second sample remain within the normal range.

Cachexia is a complex metabolic syndrome that occurs in chronic disease such as cancer (Tisdale, Physiol Rev. 2009). It has been shown in weight-losing patients that measurement of insulin response to the glucose tolerance test might be indicative of insulin resistance in the case of high insulin/glucose ratio (I/G index) or of decreased insulin secretion by β pancreatic cells in the case of low I/G index (Rofe et al, Anticancer Res 1994).

Consequently, the present Inventors measured the I/G index in cancer patients and in normal subjects. They compared the curve characterizing cancer patients with the curve of normal subjects and found at the intersection point of the two curves the existence of a corresponding critical value of MG, thereafter referred to as "cachexia-related MG control value", above which in comparison with normal subjects there is a decrease in the I/G index. This means that patients having MG production levels above the cachexia-related MG control value have a decreased insulin pancreatic secretion and therefore are entering severe pre-cachexia or cachexia.

In the context of the disclosure the cachexia-related MG control value in the blood of cancer patients is of 0.2 µM, that is about 3 fold higher than the MG normal control value in healthy subjects (see above), meaning that at the 0.2 µM MG value, cancer patient have exactly the same Insulin/glucose ratio as the one measured in healthy subjects and consequently have an identical level of insulin resistance and pancreatic secretion.

In the context of the disclosure it is said that a patient has a "high risk to develop a cachectic syndrome" (typically between 50% - 80% risk) when the MG production level in the blood is about 2 fold higher than the cachexia-related MG control value of 0.2 µM, while when the MG blood level is about 3 fold higher than said cachexia- related MG control value, the risk of developing cachexia is higher (typically between 80 - 100% risk) In contrast, it is said that a patient has a "low risk of developing a cachectic syndrome" (typically between 20% - 50% risk), when the MG blood level is about 2 fold lower than the said cachexia- related MG control value of 0.2 µM while the risk of developing a cachectic syndrome is even lower (typically between 0 - 20% risk) when the MG blood level is about 3 fold lower than said cachexia- related MG control value.

As used herein, the terms "correlation", "correlate" or "correlate with" and the like refer to a statistical association between two variables, composed of numbers, data sets and the like. A positive correlation (or "positively correlated") means that as one variable increases, the other increases as well. By contrast a negative correlation (or "negatively" or "inversely correlated") means that as one variable increases the other decreases. The present invention uses the guidelines of the US National Cancer Institute-European Organization for Research and Treatment of Cancer (NCI-EORTC) for tumor marker studies, adapted to the biochemical characteristics and biological properties of MG. NCI-EORTC Guidelines include relevant recommendations about study design, a priori hypotheses, patient an specimen characteristics, assay methods and statistical analysis. In addition, for early detection and screening perspectives, the recommendations of the NCI Early Detection Research Network (EDRN), for biomarker development were used.

It is to be considered that this invention is not restricted to particular embodiments described. It must be also considered that the terminology used herein is for the purpose of describing particular embodiments only, and is not intended to be limiting, as the scope of the present invention is limited only by the appended claims.

### DETAILED DESCRIPTION OF THE INVENTION

Methylglyoxal (MG) - the aldehyde form of pyruvic acid, also called pyruvaldehyde or 2-oxopropanal, with the formula: (CH3-CO- CH=O or C₃H₄O₂) - is a unique but ubiquitous molecule present in most biological systems including all mammalian cells (Inoue, Adv Microb Physio 1995). It is a highly reactive and dose-dependent cytotoxic metabolite that is primarily produced during glycolysis, a key metabolic step for respiring organisms.

A major discovery that distinguishes cancer cells from normal cells is that many cancer cells mainly use glycolysis in their cytoplasm to generate adenosine triphosphate (ATP) to provide cells with energy. This phenomenon of so-called aerobic glycolysis relates to the Warburg effect which is a hallmark of cancer cell metabolism (Hsu and Sabatini, Cell 2008). This effect is now well understood since it has been clearly established that cancer cells are associated with mitochondrial dysfunction and mutations in mitochondrial DNA (mt DNA) (Copeland et al, Cancer Invest 2002; Wallace, Cold Spring Harb Symp Quant Biol 2005). Excessive glycation of mitochondrial proteins, lipids and mtDNA, due to mitochondria-associated carbonyl stress have been shown to contribute to mitochondrial dysfunction, and mtDNA mutations (Pun and Murphy, Int J Cell Biol 2012). In addition, the production of free radicals in excess in the vicinity of mtDNA by dys-functioning mitochondria and the absence of protective histones in mtDNA (Baynes, Ann N Y Acad Sci 2002) may explain why the mitochondrial genome is much more susceptible both to carbonyl stress and oxidative stress than the nuclear genome and thus undergoes a higher rate of mutations (Yakes and Van Houten, PNAS 1997). Moreover it has been shown that epigenetic and/or mutagenic changes in cancer cells can induce: (1) overexpression of type 2 hexokinase (Goel et al, J Biol Chem 2003); (2) activation of normally insulin-regulated glucose membrane receptors, especially GLUT1, GLUT3 and GLUT5 (Merral et al, Cell Signal 1993), leading extracellular glucose to penetrate easily into cancer cells; and finally (3) overexpression of all glycolytic enzymes in aerobic and anaerobic conditions, causing intracellular glucose to be actively metabolized by cancer cells whatever the intra-tumoral oxygenic conditions are (Hanahan and Weinberg, Cell 2011).

The present invention is directed on the fact that cancer cells would produce characteristically significant higher amounts of MG than normal cells; making MG a potential metabolic marker of cancer. Moreover, due to both its reactive aldehyde and ketone groups, MG has been shown to be a powerful electron acceptor, and so is an extremely reactive compound characterized by unique chemical and biological properties.

In many organisms including bacteria, MG is formed as a side- product of several metabolic pathways. It may be formed from 3- amino acetone, which is an intermediate of threonine catabolism, as well as through lipid peroxidation. However, the most important source is glycolysis, wherein MG is generated through the non-enzymatic elimination of phosphate from dihydroxyacetone-phosphate (DHAP) and glyceraldehyde-3-phopshate (G-3P).

Since MG is highly cytotoxic, organisms have developed several detoxification mechanisms. One of these is the glyoxalase system, which plays a crucial role in protecting cells against electrophilic toxicity, particularly against MG-induced damaging glycation. During this process, MG activates glyoxalase 1 (GLO-1) which uses reduced glutathione (GSH) as cofactor to convert MG into S-D-Lactoylglutathione (S-D-lactoylGSH), a metabolic intermediate that is further degraded by glyoxalase 2 (GLO-2) into D-lactate (Thornalley, Gen Pharmacol 1996). Of note, the GLO-1 activity when compared to normal tissues has been shown to be increased in many human cancers, including colon, lung, breast, ovary, prostate, bladder, kidney, pancreas and stomach cancers and in leukemia and melanoma and more particularly in aggressive cancers (Jones et al, Proteomics 2002; Zhang et al, Mol Cell Proteomics 2005). Moreover overexpression of GLO-1 and GLO-2 has been correlated with multidrug resistance in tumors (Sakamoto et al, Blood 2000). However GLO-2 activity is generally lower in cancerous tissues than in normal tissues suggesting that in comparison with normal cells cancer cells could be spontaneously less capable of detoxifying intracellular MG and recovering normal GSH. This could increase both carbonyl stress and oxidative stress, hence either tumor promotion and progression or apoptosis/necrosis, depending on the intracellular free radical concentration (Irigaray and Belpomme, Carcinogenesis 2010).

A role of MG as a signaling molecule has been described. Együd and Szent-Györgyi first suggested that GLO-1 and its substrate MG are involved in the regulation of cell division (Együd and Szent- Györgyi, PNAS 1966). More recently MG has been suggested to regulate activity of the transcription factor NF-kB, and NF-kB-induced reporter gene expression (Ranganathan et al, Gene 1999; Laga et al). Moreover formation of Advanced Glycation Endproducts (AGEs) have been shown to contribute to aging and possibly to the development of general pathological conditions, such as diabetes (Brownlee, Nature 2001; Brownlee, Diabetes 2005), arterial hypertension (Wang et al, J Hypertens 2005), overweight/obesity-related adipocyte proliferation (Jia et al, PloS One 2012), Alzheimer disease (Smith et al, PNAS 1994) and cancer (van Heijst et al, Ann N YAcad Sci 2005).

Intracellular MG formation is increased under hyperglycemic conditions. Abnormal increased blood levels of extracellular MG have been evidenced in patients with types 1 & 2 diabetes (Beisswenger et al, Diabetes 1999) and recently, a mechanism by which MG can induce insulin resistance in type 2 diabetes has been described (Ribouley- Chavey et al, Diabetes 2006).

Some data clearly indicate that due to its powerful electron acceptor capacity, MG is a powerful glycating agent and the most reactive AGE precursor (Shinohara et al, J Clin Invest 1998). Not only proteins but also lipids and nucleic acids are susceptible to glycation by MG (Thornalley, Drug Metabol Drug Interact 2008).

Therefore, on the one hand MG is thought to contribute to cancer as potent mutagen and might be responsible for cancer genesis and development. On the other hand, due to its pro-apoptotic and/or pro-necrotic dose-related cytotoxic properties, it has also been thought to be an anticancer drug and believed to provide some carcinostatic effects in cancerous animals (Conroy, Ciba Found Symp 1978) and individuals (Talukdar et al, Drug Metab Drug Interact 2008). Moreover on the basis of a possible anti-tumoral effect of MG several MG-related compounds such as the compound methylglyoxal-bis cyclopentyl amidino hydrazine and the compound Mitoguazone, i.e. methylglyoxal-bis(butylaminohydrazone), commercialized under the name of methyl-GAG® (NSC-32946) have been synthesized in order to treat cancer. However neither MG nor these synthetic compounds have been demonstrated to have actually relevant anti-tumoral beneficial effects through adequate phase I and II clinical trials. Despite advances in understanding the systemic effects of MG, much remains unknown. In large part, this is because MG exists mainly adducted, given that due to its extremely high glycating properties, it bounds to intra-cellular and extracellular ligands (Chaplen et al, PNAS 1998). Further complicating the issue is that MG interacts reversibly or irreversibly with these ligands. However, it has been shown that free circulating MG can be detected in blood samples obtained from patients suffering from type 1 or type 2 diabetes (Beisswenger et al, Diabetes 1999.).

In 1959 Lewis, Majane and Weinhouse using the method of Neuberg and Strauss clearly suggested that the detection of MG in cancer cells is negligible (Lewis et al, Cancer Res 1959).

Moreover in 1978 Brandt and Siegel speculated that direct determination of MG in biological tissues is difficult because of the active glyoxalase system and thus proposed to dose D-Lactate instead of MG in the blood (Brandt and Siegel, Ciba Found Symp 1978). More recently it was concluded from a limited series of investigated patients with so called established malignant tumor, that MG blood levels were significantly decreased in breast and prostate cancer patients (Kumar, Biswas et al. Biomedical Res 2011); while it was said that MG blood levels were increased in oral precancerous lesions, i.e. in oral lesions which were said not to be established as malignant cancer. In fact, at that time, it was not clear whether the increased MG blood levels in patients with oral precancerous lesions were not due to tobacco smoking and/or alcoholism addictions, given that these risk factors are commonly associated with such subjects and that cigarette smokes as well as alcohol have been proved to contain MG (Nagao et al, Environ Health Perspect 1986); and whether patients with claimed established cancer have been or not previously treated by anti-cancer treatments and therefore whether these patients were associated or not with true clinically and/or biologically active proliferative tumor at the time of blood sample collection.

The Inventors surprisingly found that the blood levels of MG are significantly elevated in patients suffering from established progressive cancers, whereas in non-metabolically active cancers, i.e. in precancerous states or even in in situ stage 0 cancer MG blood levels is not significantly elevated. Indeed, MG blood levels are significantly increased in epithelial cancer such as head and neck cancers, lung, breast, prostate, colorectal, pancreas and other digestive cancers; and in non-epithelial cancer such as leukemia, lymphoma, melanoma and sarcoma. More precisely, the MG blood levels correlate with the tumor volume and therapeutic responses in cancer suffering patients. The higher the MG blood level is, the higher the tumor burden. MG level therefore and critically appear to be a clinically meaningful biomarker to aid oncologists in decision making and treating cancer patients.

Accordingly, the present disclosure relates to MG for its use as a clinically useful biomarker for monitoring in cancer patients, human or animals. As MG blood levels can be precisely and rapidly measured, the diagnosis method of the disclosure contributes to disease monitoring and therapeutic response assessment in a very sensitive manner. Finally, since MG production by cancer cells relates to a fundamental and characteristic metabolic dysfunction of these cells the use of MG as a biomarker of cancer allows for the detection of many, if not all cancer; in contrast with the presently available type-related tumor biomarkers. Another object of the disclosure is a kit for monitoring anticancer therapeutic response.

Another object of the disclosure is the use of MG in the early detection and diagnosis of metabolically active cancer measuring and analyzing the production of MG in samples of extracellular fluids, cells and/or tissues by using any chemical or immunological *in vitro* method of MG measurement; given that the use of MALDI-TOF/TOF mass spectrometry or similar techniques are preferred.

### 1. MG as a natural intra-tumoral biomarker produced by cancer cells.

The inventors found that cancer cells can produce and release higher amount of MG than normal cells, that cancer cells produce and release large amount of MG directly within the tumor, then in the extracellular compartment in the organism and more particularly in the peripheral blood; whereas normal cells (or inflammatory cells) produce and release no or only low detectable amount of MG in tissues and in the extracellular compartment in the organism, more particularly in the peripheral blood.

These surprising results have been confirmed in *in vitro* cultures, and animal models and more particularly by using MALDI-TOF/TOF mass spectrometry for in situ MG tumoral analysis, and clinically with patients. MG can be directly detected in tumor tissues and the tumor area where MG is detected mostly corresponds to the active proliferation zones in the tumor (see Fig 2). Moreover, the amount of MG produced and released by cancer cells in cultures depend on the glucose concentration in the culture medium i.e. the higher the glucose concentration, the higher is the MG production by cancer cells (see Fig 1 bis), confirming that cancer cells mainly use glycolysis for ATP production, even in aerobic conditions. In addition the inventors have shown that the amount of MG synthesized and released from the tumor is positively correlated with the tumor burden, i.e. the higher the tumor volume, the higher is the MG production level in the peripheral blood (see Fig 3 for cancer patients and Fig 5 for animal model); whereas in the case of a tumor rejection by inflammatory and/or immune competent cells, MG levels remain very low (see Fig 6). Consequently, one major embodiment of the invention is that the MG production level detected in the tumor and/or in the extracellular compartment in the organism of a cancer-bearing subject relates to the level of metabolic activity of cancer cells, which corresponds to the level of proliferative activity of the tumor from which the subject is suffering.

The present disclosure is therefore drawn on a method for the early detection and diagnosis of cancer by measuring and analyzing the in situ production of MG by metabolically active cancer cells in samples of cells and/or of tissues, by using any chemical or immunological *in vitro* methods of MG measurement. These methods include the use of MALDI-TOF/TOF mass spectrometry or similar techniques.

Consequently the present disclosure encompasses MG for its use in a method for detecting cancer by measuring and analyzing the production and release of MG in tissue and/or cell samples using tissue biopsies, as it is commonly done for any solid tumor and/or any cellular smears, as is commonly used for hematological cancer diagnosis and monitoring (leukemia, lymphoma) and/or for screening of some solid cancer (uterus cervix) as well as other cancer types. Because the major part of MG that is produced and released from cancer cells comes from their increased glycolytic activity, the present disclosure also encompasses a method for determining the proliferative aggressiveness of a tumor and thus may contribute to distinguish cancer from benign tumors, or inflammatory processes since the metabolic activity of cancer cells is generally enhanced in comparison with that of cells of benign tumors or inflammatory cells.

### 2. MG as a natural biomarker released by cancer cells in extracellular fluids for early detection, diagnosis and prognostic evaluation in non-diabetic subjects.

In a second major example, the disclosure encompasses a method for determining the existence of a tumor in said subjects, by measuring MG production levels in biological samples of the extracellular compartment in the organism; more preferably in the peripheral blood; and comparing the measured MG production level to their normal control value.

The present disclosure is also drawn to an *in vitro* method for early detection, screening and diagnosis of cancer in non-diabetic subjects, comprising the steps of:
a) determining the production level of MG in a biological sample of said subjects from an extracellular fluid,
b) comparing said MG production level to a control value, i.e. to the MG level in non-cancer subjects wherein if the MG production level in said biological sample is higher than the said control value, the said subjects are suffering from cancer or have a high risk of having it.

In contrast, when the MG production level in said biological sample is within the range of the said normal control value, said subjects are not suffering from cancer or have a low risk of having it. The present disclosure enables the detection and diagnosis of cancer in human or animal subjects who are non-diabetic, i.e., in subjects having a level of glycated hemoglobin HbAlc below 7%. In a preferred embodiment, the diagnosis method of the disclosure enables detection of head & neck, bronchus & lung, breast, prostate, colorectal, pancreatic and other digestive tract cancers, in addition to ovarian & endometrial, renal & bladder cancers, leukemia and non-Hodgkin lymphoma, melanoma and sarcoma. In a preferred embodiment, the MG normal control value is the production level of said MG which has been measured in a biological sample of healthy individuals. Preferably, this value for whole blood is 0.06 µM ± 0.02 with a confidence interval of 0.02 µM to 0.11 µM. Moreover the present disclosure also encompasses a method for determining the proliferative aggressiveness of a tumor comprising the step of measuring MG in a biological sample of said subjects and comparing the measured MG production level to its control value.

### 3. Early detection and diagnosis of cancer: Diabetic patients.

There is a statistically significant higher incidence of cancer in non-treated type 1 and type 2 diabetes mellitus patients. However, the production level of MG is known to be increased under hyperglycemic conditions, i.e. in non-treated or not correctly-treated diabetics, (McLellan, Clin Sci 1994). Therefore the present MG cancer biomarker would be confounded in these patients.

An object of the disclosure is therefore that the MG/G index enables discriminating those patients who may have a cancer from those who may not, even in diabetic patients. The evaluation of this index therefore enables the early detection and diagnosis of cancer in diabetic patients and consequently will improve cancer prognosis in these patients.

The present disclosure is therefore drawn to an *in vitro* method for early detecting, screening and diagnosing cancer in diabetic subjects, comprising the steps of:
a) determining the production level of MG in a first biological sample of said diabetic subjects,
b) determining the glucose level in a second biological sample of said subjects,
c) comparing the MG/G ratio of these two levels (MG/G index) to corresponding control ratio determined in healthy individuals and normo-glycemic treated diabetic subjects, wherein if the MG/G index obtained in step c) is higher than said corresponding control ratio, said subjects are considered suffering from cancer or to be at increased risk of cancer; wherein if the MG/G index obtained in step c) is similar to said corresponding control ratio, said subjects are considered neither to be suffering from cancer nor to be at increased risk of cancer.

Importantly, this method can be applied to any non-diabetic animal or human subject, preferably to correctly but even to incorrectly treated diabetic patients, i.e. to subjects with a level of glycated hemoglobin HbAlc lower to 7%.

As mentioned previously, the said first and said second biological samples (which are devoted to the measurement in the same individual of MG and glucose levels, respectively) are preferably samples of biological fluids, for example chosen from blood, serum, plasma. In the method of the invention, said first and second samples must be of the same nature (i.e., both be blood etc.).

The said first and said second samples can be collected sequentially from the subject. In the preferred embodiment, the samples are collected at the same time. In a better embodiment, the one sample is split into two, so that the levels of MG and glucose are measured in the same sample. Several methods are routinely used to measure glucose level in a biological sample. The skilled person well knows how to measure glucose level depending on the type of the biological sample. For example, when a blood sample is used, glucose can be measured on whole blood, plasma or serum by routine techniques. However the sample has to be kept at 4°C if MG is to be reliably measured.

But, in the context of the disclosure, electrical or enzymatic glucose measuring techniques are preferred. The two most common employed enzymes are glucose oxidase and hexokinase. In a preferred embodiment, glucose is measured by measuring the level of hydrogen peroxide (H2O2) formed when glucose reacts with glucose oxidase.

The level of MG is measured in the biological sample, as disclosed previously.

In a preferred embodiment, the MG/G index measured in and determined from a biological sample of healthy individuals or of normo-glycemic treated diabetic subjects is preferably a value of about 0.01 µmoles/g, corresponding to a MG/G index value that is intermediate between the median MG/G index value obtained from the blood of healthy donors and the median MG/G index value obtained from the blood of non cancerous normo-glycemic treated diabetic patients (Fig 4).

### 4. Staging, prognostic assessment, monitoring and therapeutic evaluation in cancer patients

Imaging techniques are not accurate to detect initial cancerous states as well as to correctly stage cancer into the four internationally recognized (I to IV) categories. Indeed, a critical concern for clinical oncologists is to evaluate correctly cancer progression and extension through the organism during sub-clinical states.

The present disclosure shows that in animals the production levels of MG correlate with the tumoral volume (Fig 5), and that in patients MG production levels in the peripheral blood correlate with the stage of the tumors (Fig 3) and with the tumoral response after treatment (Table 3).

The present disclosure is therefore drawn to an *in vitro* method for staging disease and prognostic evaluation in cancer patients, whether human or animal, by determining the production level of MG in a biological sample, preferably a blood sample obtained from said patients and for monitoring therapeutic efficiency in cancer patients, comprising the steps of:
- for staging and prognostic evaluation:
   a) determining the MG production level in an initial pretreatment biological sample obtained from said patients,
   b) comparing said pretreatment MG level to normal MG control value,
   c) classifying said pretreatment MG level according to one of the four stages of staging classification,
- for monitoring efficiency of anti-cancer treatment:
   a) determining an initial pretreatment MG production level in a first biological sample obtained from said patients,
   b) determining a second MG production level in a second biological sample obtained after treatment from said patients,
      wherein said second sample is obtained at a given time after obtaining the first sample,
   c) comparing said initial and said second MG production
   levels, wherein if said second MG production level is higher than said initial MG production level, said treatment is considered not to be efficient on said patients; whereas if said second MG production level is lower than said initial MG production level, said treatment is considered to be efficient on said patients and should be preferentially pursued.

This monitoring method can be applied to any human or animal subjects presenting with a cancer.

The present *in vitro* method can also be used for monitoring the therapeutic efficiency of any prophylactic anticancer treatment administered to non-symptomatic subjects whose sub-clinical cancer has been detected by using the present method.

The present *in vitro* method can also be used for monitoring the therapeutic efficiency of any prophylactic anticancer treatment administered to cancer patients already treated for a perceptible disease, and for whom adjuvant anticancer treatment is required to treat a residual subclinical disease, preferably by using the present method.

As mentioned previously, the first and second biological samples (i.e. respectively the pre- and post-treatment samples) are preferably samples of biological fluid, for example chosen from whole blood, serum, plasma and should otherwise be as identical as possible. In this method, said first and said second samples have to be collected in a staggered manner, so that the said anti-cancer treatment performed in-between sample collection has had sufficient time to develop its efficacy and the result obtained be measured and interpreted according to the present disclosure. More precisely, as indicated above said second biological sample has to be obtained "at a given time after the first sample", that is, depending on the growth doubling time of the cancer, at least one month; preferably two or three months or even six months after the first sample; and preferably after the said treatment has been given entirely or has been initiated during a sufficient long time to provide interpretable results.

The present disclosure is also drawn to an *in vitro* method for predicting the survival chance of a patient suffering from cancer, via a biological sample of said patients, comprising the steps of:
a) determining an initial production level of MG in a first biological sample obtained from said patients,
b) determining a second production level of MG in a second
   biological sample obtained from said patients,
   wherein said second sample is obtained at a given time after
   obtaining the first sample,
c) comparing said initial and second production levels,
wherein if said second MG production level is higher than said initial MG production level, the said patients are predicted to have a short-term survival chance; wherein if said second production level is lower than said initial production level, the said patients are predicted to have a prolonged survival chance.

This therapy efficacy-prediction method can be applied to any human or animal subject presenting with a cancer.

As indicated previously, the first and second biological samples (i.e. respectively the pre- and post-treatment samples) are preferably samples of biological fluid; for example chosen from whole blood, serum, plasma and must be of the same nature. Again, in this method, said first and said second samples should be collected sequentially, that is, one month, two months, three months or even six months after the first sample, depending on the growth doubling time of the cancer the shorter the growth doubling time is, the shorter the time interval should be.

Preferably, the method is performed on a blood sample.

Of note, when the MG production level in said second sample is similar to the MG production level in said first sample; i.e., if their ratio is comprised between 0.7 and 1.3, and even more preferably between 0.9 and 1.1, said first and second samples being collected for example at one-month interval, then it is not possible to predict precisely if the patient has a growing, stable or collapsing cancer. It is thus necessary to proceed with the same treatment and to repeat the measure weeks or months later on to confirm the result.

### 5. Prediction and early detection of cachexia

Cachexia is estimated to occur in a large percentage of patients with cancer (especially in those with cancers of the pancreas, stomach, colon and lung) and is associated with poor quality of life and reduced survival time, irrespective of tumor burden and the presence of metastasis. It is characterized clinically by reduced food intake and weight loss, and biologically by systemic inflammation, increased lipid mobilization and oxidation, increased whole body protein breakdown and turnover, and impaired carbohydrate metabolism. In cachectic patients, alterations of carbohydrate metabolism include glucose intolerance, whole body insulin resistance, decrease host glucose oxidation, increased glucose neo-genesis and increased glucose turnover and recycling; all processes in which insulin plays a key role (Tayek, J Am Coll Nutr 1992).

The present Inventors measured MG in relation to the BMI and found that MG production blood levels are significantly inversely correlated with the BMI of cancer patients but not with the BMI of normal subjects (see Fig 7); and that in cancer patients with a BMI lower than 18, i.e. in patients with a pre-cachectic or cachectic syndrome, MG production levels are significantly increased in comparison with non cachectic cancer patients (Table 4). This means that measuring MG in cancer patients may be a valuable tool for predicting or confirming a diagnostic of cachexia. Moreover, in cachectic cancer patients, glucose intolerance and more specifically insulin resistance are early biochemical events, occurring long before the onset of weight loss (Tayek et al, J Am Coll Nutr 1992) and in weight-losing patients, measurement of insulin reponse to the glucose test tolerance might be indicative of insulin resistance in the case of high Insulin/Glucose (I/G) index or decreased insulin secretion in the case of low I/G index (Rofe et al, Anticancer Res 1994). Consequently the present inventors measured the I/G index in cancer patients and in normal subjects according to the MG production level and established that the cachexia-related MG control value in the blood of cancer patients is 0.2 µM, meaning that at the 0.2 µM MG value cancer patients have exactly the same I/G ratio as the one measured in healthy subjects, and that consequently they have an identical level of insulin resistance and pancreatic secretion (see Fig 8).

Thus, the present disclosure is also drawn to an *in vitro* method for predicting, detecting and diagnosing cachexia or pre-cachexia in cancer subjects or patients comprising the steps of:
a) determining the MG production level in a biological sample obtained from said patient,
b) comparing said MG production level to a cachexia MG- related control value,
wherein if the MG production level in said biological sample is higher than the said cachexia MG-related control value, then the said patient is entering cachexia or severe precachexia and therefore in the absence of efficient specific anti-cachectic treatment are predicted to have a short-term survival
whereas if the MG production level in said biological sample is lower than the said control value the said patient is not entering cachexia or severe precachexia and therefore are predicted to have a more prolonged survival.

In this method, the said MG control value has been determined from a comparison between the evolution of the insulin/glucose ratio (I/G index) in cancer patients and the evolution of the I/G index in normal subjects, allowing the characterization of a critical point of MG production level termed "cachexia-related control value", estimated to be about 0.2 µM MG in the blood and above which the level of insulin secretion by β pancreatic cells is deficient meaning that cancer patients enter cachexia or severe precachexia.

That is about 3 fold higher than the MG normal control value in healthy subjects.

Once again, this predicting method can be applied to any human or animal subjects who present cancer.

### METHODS OF METHYLGLYOXAL MEASUREMENT

A direct *in situ* analysis/detection of MG in samples of solid tissues and more particularly of tumors can be made by using MALDI- TOF/TOF mass spectrometry, which associates a matrix assisted laser desorption/ionization (MALDI) with a time-of-flight mass spectrometry (TOF).

The procedure carried out for direct in situ measurement and analysis of MG in solid tissue biopsies and cellular smears by MALDI-TOF/TOF mass spectrometry is decribed below in "Examples". Briefly as far as solid tissues are concerned, prior to be cut into 12 µm thickness slices, solid tissues are firstly frozen at - 80°C and fixed by ultrapure water during the cryostat procedure. Slices are then put on specific MALDI plates and treated with ethanol before being treated with alpha-phenylene diamine (o-PD). Preparations are thereafter incubated in a humidified chamber overnight at room temperature in the dark, then dried (using a desiccator) and coated with α-Cyano-4-hydroxycinnamic acid (HCCA) matrix solution. By analyzing the effect provided by MALDI-TOF/TOF mass spectrometry on 2MQX, the Inventors discovered that the two 2MQX molecular fragments, one of 91 Da and the other of 118 Da are the best selected signature of MG that could be used to detect and quantify MG in solid tissues, after using MS/MS imaging analysis. A similar procedure as been set up and carried out for detecting and measuring intracellular MG in cell smears. The analysis / detection of free MG in liquid samples can be performed by conventional means known in the art, for example by using reverse phase high performance liquid chromatography (RP- HPLC), ELISA tests, or other methods that have been proposed (see Ohmori et al, J Chromatogr. 1987; McLellan et al, Anal Biochem 1992; Nemet et al, Clin Biochem 2004; Chaplen et al, Anal Biochem 1996).

In a preferred embodiment of the invention, said fluid biological samples are chosen from whole blood, serum, plasma. In a preferred embodiment of the invention, detection of naturally occurring free MG is performed by adding to the blood sample a 1,2-diaminobenzene derivative, preferably o-phenylene diamine (o-PD). The reaction between MG and o-PD indeed forms quinoxalines, which are strong chromophores or fluorophores or both, easily quantified with RP-HPLC. However the invention also uses 1,2-diamino-4,5-dimethoxybenzene (DMB also called DDB) according to the method described by McLellan et al. (McLellan et al, Anal Biochem 1992) which measures the resulting quinoxaline also by RP-HPLC.

A simple method for quantifying level of MG in the whole blood sample is provided in the experimental part below. In this particular embodiment, the whole blood sample is collected from the subject by conventional means, and immediately kept on ice before being frozen at -80°C until MG is measured. After defrosting, up to the temperature of the derivatization procedure the sample is kept at 4°C, as MG is very reactive and unstable. In a first step, trifluoroacetic acid (TFA) is added to the defrosted whole blood sample for instantaneously protein precipitation. The sample is thereafter centrifuged at 4°C and the supernatant is recovered. In a second step, derivatization is performed by adding o-PD or DMB to the supernatant, and said mixture is kept for 4-6 hours at room temperature (23°C) in darknes. A final centrifugation is performed and the supernatant recovered, so as to be analyzed using RP- HPLC or gas chromatography, coupled with detection system, both which precisely quantify levels of MG.

Alternatively to this procedure, the Inventors propose an improved method for simplifying sample collection and treatment and MG measurement. In this alternative method, vials already containing TFA are used for sample collection, samples are immediately mixed by inversion and kept at 4°C before being frozen at -80°C. So after defrosting, sample can be immediately centrifugated at 4°C and supernatant obtained, derivatized for MG quantification as above.

MG measurement can also be done by using a quantitative "sandwich" enzyme-linked immunosorbent assay ("sandwich" ELISA) based on the preparation of specific antibodies against MG. Preparation of antibodies specific to free MG is crucial for the validity of this test. Several human MG ELISA kits are commercialized.

In a preferred embodiment of the disclosure, antibodies specific to MG are pre-coated onto microplates. Calibrated samples are then introduced into the pre-coated microplate wells, so free MG that is present in the sample binds to pre-coated antibodies. After removing any unbound substances HorseRadish Peroxidase (HRP)-conjugated anti-MG antibodies are then added directly to the wells. After washing this is followed by the addition of 3,3',5,5' tetramethyl-benzidine (TMB) substrate solution (a specific substrate for the enzyme conjugate used) to each well. Only the wells that contain MG will evidence a change in color that can be measured by spectrophotometry. Finally MG levels in the samples are determined by comparison with a standard. This quantitative "sandwich" enzyme immunoassay is a simplification of the available commercialized ELISA tests, using for example a system of biotin-conjugated antibodies coupled with avidin-conjugated HRP. Since the validity of "sandwich" ELISA tests will depend on the specificity and quality of the anti-MG antibodies, such tests should involve regularly RP-HPLC control checks of each new stock of reagents.

### REDUCING FALSE NEGATIVE AND FALSE POSITIVE RESULTS

From the data presented herein (see Fig 3 and "Examples") when measuring MG in the whole blood of cancer patient by RP-HPLC the Inventors evaluated the possibility of false negative results of 10 to 15 % of the time. In such cases other methods such as those of the invention which measure directly MG in tissues or cells have to be employed. False positive error may occur with chronic uremia (Nakayama et al, Am J Nephrol 2008) and types 1 & 2 diabetes mellitus patients, but chronic uremia and diabetes can be easily recognized and diagnosed, and the Inventors have proposed the use of an MG/G index to detect cancer in diabetic patients. As indicated previously, in addition to diabetes, AGEs have been associated with aging and several non cancerous age-related diseases such as arterial hypertension, overweight/obesity and Alzheimer disease. An increase in MG levels has been detected in the arterial wall and in the blood of hypertensive rats (Wu and Juurlink Hypertension 2002) but it has never been proved that patients with common arterial hypertension have increased MG production levels in their blood. It has been reported increased protein glycation and MG levels in the cerebrospinal fluid of patients with Alzheimer's disease, but MG has not been observed increased in the peripheral blood of the patients. Moreover the advanced glycation end product-associated parameters detected in the peripheral blood of patients with Alzheimer's disease were found to be of lower values in comparison with non-demented controls (Thorne J et al, Life Science 1996), a finding that does not suggest that MG blood levels might be increased in such patients. Indeed, with the exception of chronic uremia and types 1 & 2 diabetes mellitus, there is no data supporting the presence of high blood levels of free MG in humans with age-related diseases such as arterial hypertension or Alzheimer disease. Moreover in normal healthy subjects, aging was not considered to influence blood MG production levels and age-related MG blood levels was included within the normal range values, so aging by itself could not constitute false positivity. In addition, any increase in MG production levels has not been observed in the blood of several patients with chronic inflammatory disease.

In another aspect, the present disclosure is drawn to a kit for monitoring anticancer therapeutic response comprising:
- the means for collecting biological samples,
- the means for measuring MG production levels,
- the instructions for using said kit,
- optimally, a control (reference) sample.

In a preferred embodiment of the disclosure the said kit comprises instructions and means for in situ detecting and measuring MG in cell smears or tissues by MALDI-TOF/TOF mass spectrometry or similar techniques and quantifying MG, by using one of the available methods:
- A chemical test, including o-PD or DMB, 2MQX or DMQ, MQX or DDQ for RP-HPLC analysis in extracellular fluids

For the chemical test, the said kit comprises the following reagents:
- Trifluoroacetic acid (TFA) for protein precipitation
- o-phenylenediamine (o-PD) or 1,2-diamino-4,5- dimethoxybenzene (DMB also called DDB) for derivatization
- The specific quinoxaline product corresponding to the derivatizing agents used: 2-methylquinoxaline (2-MQX) or 6,7-dimethoxy-2-methylquinoxaline (DMQ) for the calibration curve.
- Standards consisting of the quinoxaline derivatives 5- methylquinoxaline (5-MQX) or 6,7-dimethoxy-2,3-dimethyl-quinoxaline (DDQ) for internal standard.

- Optionally, a chemical test using chemical reagents for MALDI-TOF/TOF mass spectrometry analysis for MG measurement in solid tissues or cell smears.
- Optionally a quantitative "sandwich" enzymatic immunological test based on monoclonal or polyclonal antibodies recognizing specifically free MG, for MG measurement in extracellular fluids.

In another preferred embodiment, the kit of the disclosure further comprises the means for detecting glucose production level and instructions for determining the MG/G index based on the glucose oxidase or hexokinase enzymatic tests.

### Example 1: Solid tissues sample preparation and MG measurement in tumors

Tumor specimens were obtained 6 weeks after 90 male and female BD-IX rats (Charles River, France) have been grafted with PRO tumorigenic cancer colonic cells (45 females and 45 males provided from Charles River). Prior to be cut into 12 µm thickness slices, tumors were frozen at -80°C and fixed by ultrapure water during the cryostat procedure at -20°C. Slices were then put on specific MALDI plates (provided from Bruker) and the preparations were treated with ethanol, then with o- PD (0.01%) (Sigma Aldrich, France) before being incubated in a humidified chamber overnight at room temperature in the dark. After this incubation, slices were dried (using desiccator) and coated with a matrix solution of α-Cyano-4-hydroxycinnamic acid. (HCCA) (provided from Sigma Aldrich). By analyzing the effect on 2MQX (2- methylquinoxaline) (provided from Sigma Aldrich) with MALDI TOF/TOF mass spectrometry (Bruker UltraFlex III), two 2MQX molecular fragments, one of 91 Da and the other of 118 Da were selected which allowed the detection of MG in the tumor after MS/MS imaging analysis.

Control ranges were prepared as follows: the internal standard 5MQX (5-methylquinoxaline) (provided from Sigma Aldrich) was used at 0.4µM and mixed at this final concentration with each aliquot of 2MQX, prepared according to a range of concentrations, from 0 to 1.6 µM. Dilutions were done with ultrapure water. Analysis was done using MALDI-TOF/TOF mass spectrometry

### Example 2 : Extracellular fluids sample preparation and MG measurement in blood:

The subjects have to be fasted for 8-12 hours before sampling since MG may be present in some food and beverage. Blood samples are harvested at 4°C and analysis can be done on the whole blood because MG is at a constant concentration in red blood cells. This possibility derives from the fact that in red blood cells, MG is produced non-enzymatically at a constant rate from glycerone phosphate and glyceraldehyde-3-phosphate (Thornalley, Biochem 1989).

A method based on a simple derivatization procedure followed by gas chromatography/mass spectrometry (GC/MS) analysis has been used. Preparation and quantification of MG are done using a reverse- phase high performance liquid chromatography (RP-HPLC) method involving derivatization either with o-PD, or DMB coupled with mass spectrometry analysis. Briefly, after whole blood centrifugation at 4°C, the processing requires protein precipitation with trifluoroacetic acid (TFA), incubation of the supernatant with the derivatizing agent o-PD or DMB for 4-6 hours at 23°C in the dark, and quantitative analysis of MG after its conversion into 2MQX for o-PD, or 6,7-dimethoxy-2- methylquinoxaline (DMQ) for DMB.

The standard solutions are prepared as follows: The concentration of the stock aqueous solution of MG is determined enzymatically by endpoint assay. MG quantification involves conversion to S-D-lactoylglutathione by glyoxalase I in the presence of reduced glutathione (GSH). Calibrating standards containing 0.0625-1.6 nmol of MG in 1 ml of water is prepared. Derivatization is carried out by the procedure described above. Calibration curves are constructed by plotting the pick area ratios of 2MQX and 5MQX (internal standard) against the MG concentrations for the derivatizing agent o-PD or by plotting the pick area ratios of DMQ and 6,7-dimethoxy-2,3-dimethyl-quinoxaline (DDQ) (internal standard) against the MG concentrations for the derivatizing agent DMB.

In order to identify and determine the concentration of MG in blood, the quinoxaline derivatives 2MQX and 5MQX for o-PD, and DMQ and DDQ for DMB are resolved by RP-HPLC and analyzed by electrospray ionization/selected ion monitoring (ESI/SIM). Finally MG quantification is performed by calculating a peak area ratio of protonated molecular ion peak intensity (m/z 145 for 2MQX and m/z 205 for DMQ) to a protonated molecular internal standard ion peak intensity (m/z 145 for 5MQX and m/z 218 for DDQ) in the selected ion monitoring mode (SIM).

### Example 3 : In vitro experiments

Measurement of MG production by cancer cells in comparison to normal cells have been done by using *in vitro* tissue cultures. In a typical experiment using cell cultures MG production by cancer cells in the conditioned medium (CM) of the HCT116 human colorectal carcinoma cell line was done using LC-MS/MS. Cells were cultured in either low glucose condition (5.6 mM) or high glucose condition (25 mM) and collected after 48 hours.

It was found that MG production in the CM is 10 fold higher in high glucose condition (MG concentration of 0.05917 µM) than in low glucose condition (MG concentration of 0.00515 µM), showing that cancer cells synthesize MG from glucose and so mainly use glycolysis for ATP production.

Further experiments showed that this dose-dependency concerns various types of cancer cells; whereas because of lower glucose consumption and glycolysis, normal cells synthesize and release less MG.

### Example 4: In vivo experiments

A series of 101 consecutive patients with a variety of cancer types and localizations at different stages of their disease was analyzed for the presence of MG in the blood and the levels obtained in cancer patients were compared to those obtained in a series of 36 normal controls adjusted for age and sex and of 12 patients with normo-glycemic treated for type 2 diabetes mellitus (in addition to 6 non-treated type 2 diabetes mellitus used as a positive control for the test). Inclusion criteria for cancer patients were a pathological diagnosis of cancer, the absence of previous treatment, the presence of a clinically and/or biologically perceptible disease, the absence of diabetes mellitus, renal insufficiency and other chronic diseases.

Inclusion criteria for normal controls were the absence of cancer, diabetes mellitus, arterial hypertension, Alzheimer's disease and renal insufficiency; for patients with non-insulin-dependent type 2 diabetes, no diabetes-associated complications and for treated diabetic patients a glycated hemoglobin HbA1c<=7% and a normal glycemia. For all included subjects, inclusion criteria were no smoking, no alcohol and no coffee consumption 24 hours before time of sample collection and all patients with high tobacco smoking and/or alcoholism addiction were excluded from the study. The BMI as well as measurement of blood glucose and insulin were serially determined according to standard procedures in the first 66 included patients and in all controls.

### Example 5: In vivo animal models

A set of experiments was conducted using laboratory animals, in particular the model of 1-2 dimethylhydrazine-induced transplantable colonic cancer in syngeneic BDIX rats, for which two carcinoma cell clones, (DHD-K12/SRb and DH-K12/JSb) have been previously selected *in vitro* to form progressive (PROb) tumors and regressive (REGb) tumors respectively, when grafted to the rats. In these experiments, blood samples for measurement of MG and other molecules such as glucose and insulin were harvested on weeks 2, 3, 4, 6 and 9. At the same time tumor masses were measured for tumoral evaluation. Statistical analysis was performed using JMP 7 (SAS Software, NC, USA). Statistical significance was determined by using Fisher exact test and the two-tailed Student's t-test.

The colonic tumor is an adenocarcinoma that was obtained from BD-IX rats 6 weeks after transplantation of PRO tumorigenic cancer colonic cells (see above). In 1 and 2 of Fig 2, the tumor in clearly associated with a large necrotic zone that predominates in its middle and lower part. This is particularly well evidenced in 2 of Fig 2, which corresponds to the tumor stained by Hematoxilin-Eosin-Safran.

MG has been localized in the tumor by detecting the two molecular fragments of 2MQX, one of 91 Da and the other of 118 Da after MS/MS imaging analysis by MALDI-TOF/TOF. This allowed to obtain the tumoral scans that are shown in 3 and 4 of Fig 2 respectively.

Scans in 3 and 4 are examples proving that malignant tumors are capable of producing high amounts of MG, whereas normal control tissue analysis by using this method revealed no or low detectable MG. As reported in scans 3 and 4 of Fig 2, it was not clear whether MG was detected intra-cellularly, extra-cellularly or both. However in scan 3 of Fig 2 (which corresponds to the 91 Da 2MQX fragment) MG amount appears to be less abundant in the necrotic zone of the tumor, while it appears to be mostly detected in the active proliferative part of the tumor.'

### Example 6: Cancer patients

The results of Table 1 demonstrate that the mean and extreme values of MG blood levels in cancer patients are significantly higher than those in normal controls, both for male and female, and in patients with normo-glycemic treated type 2 diabetes mellitus. No significant difference between normal subjects and patients with normo-glycemic treated type 2 diabetes mellitus used as control was found.

In addition to MG blood determination, patients with pathologically proved cancer were prospectively and serially investigated for blood glucose and insulin before anticancer treatment. A similar investigation was done in normal subjects. In cancer patients, no significant correlation between MG blood levels and glycemia was found, while MG blood levels tended to be inversely correlated with insulinemia (data not shown) meaning that in cancer patients MG blood level is a relatively independent parameter. A non- significant result was found in normal controls. Such data therefore mean that detection of an increased MG blood level in correctly treated diabetic patients, i.e. in patients with normal glycemia and normalized HbA1c, could be due, as for healthy non-diabetic subjects, to cancer outcome.

Systematic measurements of MG in normo-glycemic treated diabetic patients is therefore justified as an high incidence of certain types of cancer including colo-rectum, pancreas, liver, breast, and bladder cancers, has been shown to be significantly associated with types 1 or 2 diabetes mellitus.

The results of Table 2 disclose a comparison of the MG blood levels in cancer patients according to the types of tumor:
Indeed, in comparison with normal controls (and normo- glycemic treated type 2 diabetes patients) the MG blood levels are significantly increased in patients suffering from head & neck, lung, breast, prostate, colorectal, pancreas and/or other digestive cancers and demonstrate that in these patients the different MG values are between 1,5 and 2 fold higher than the normal control value depending on the tumor type. Of note are the statistically significant MG level difference from normal controls obtained for breast and prostate cancers which are the most frequent cancers; and the highly statistically significant MG level differences for lung, colo-rectum, pancreas, and head and neck cancers, for which there are presently no available early detection biomarkers.

### Reference Example 1: Statistically significant relationship between MG levels in the blood of cancer patients and disease stages.

Since it is well demonstrated that tumoral volume is of prognostic value, MG blood levels at staging can be considered as a prognostic indicator. Moreover since the Inventors have shown that MG blood levels clearly reflect tumoral volume MG blood levels are also a prognostic indicator later on during disease evolution.

For the in situ cancers (stage 0) there is no significant increased MG blood levels in comparison with the normal control value (0.06 µM), a finding that confirms that some stage 0 cancers may not be metabolically active, while for stage I to IV cancer there is a significant positive correlation (p=0.0109). This means that systematic measurement of MG in the blood of cancer patients is an efficient tool for diagnosing and staging cancer and for prognostic assessment.

### Example 7: MG blood levels and tumoral volume in animal experiments.

Figure 5 discloses the evolution of MG blood levels in BD-IX rats after transplantation of PROb tumorigenic cancer colonic cells and Figure 6 the evolution of MG blood levels in BD-IX rats after transplantation of REGb non tumorigenic cancer colonic cells. As demonstrated from these data, there is a clearly statistically significant positive correlation between MG blood levels and the tumor volume in rats grafted with PROb tumoral cells. In contrast, in rats transplanted with REGb tumor cells and for which the graft cannot take, the MG blood levels, after a transient increase at week 4 after transplantation could not be further detected, meaning that, in animals for which the tumoral graft did not take, no significant increased amount of circulating MG was evidenced. This experiment shows that growing tumors are significantly associated with higher MG blood levels than non-growing tumors, i.e. that proliferative cancer cells produce and release higher circulating MG amounts than non-proliferative cancer or normal cells. This explains why increased MG blood levels are detectable in cancer- patients, whereas significant lower MG blood levels or even no MG blood levels are detected in subjects with no cancer, more precisely with no proliferative active cancer.

### Example 8: Mean and extreme values (in µM) of MG blood levels according to clinical responses obtained in treated cancer patients.

As indicated in Table 3, longitudinal studies of several patients treated for cancer showed that patients who were evaluated clinically for complete response after anticancer treatments were associated with normal MG blood levels, whereas patients who failed to respond to treatment or had a partial response or a stable disease after treatment had persisting high MG blood levels. Thus in cancer patients MG is a marker of disease evolution and therapeutic response. However, several patients who were considered to respond completely to treatment by using the presently available biomarkers and imaging techniques for evaluating response did have still detectable increased levels of MG in their blood, which levels were further associated with early tumoral relapse. This finding strongly suggests that MG detection in treated cancer patients may be a better tool for the evaluation of tumoral therapeutic response than the currently available clinical approaches based on classical biomarkers and/or imaging techniques.

### Reference Example 2: MG/G index in the blood of cancer patients, normal subjects and treated type 2 diabetes patients.

As shown on Figure 4, the MG/G index determined in blood is significantly increased almost two fold in cancer patients in comparison with the one in healthy subjects and normo-glycemic treated type 2 diabetic patients. This result strongly suggests that it is possible to recognize diabetic patients with cancer (having a high MG/G index) from those who are not with cancer (having a low MG/G index); despite the diabetic's potentially MG-confounding glucose dys-regulation.

### Reference Example 3: Correlation between MG blood levels and BMI in cancer patients and healthy subjects.

As it has been shown that patients with overweight/obesity are associated with a significant increase in cancer incidence, search for a correlation between MG blood levels and BMI was performed in cancer patients versus normal controls.

### a. MG blood levels in cancer patients with overweight-obesity

As displayed in Table 4 cancer patients with overweight/obesity (BMI>25) are associated with lower - but still high - MG blood levels in comparison with cancer patients having a normal weight (18<BMI<25). However, unlike in normal subjects, there is in cancer patients a statistically significant inverse correlation between BMI and MG blood levels (Fig 7), meaning that detection of an MG blood level higher than 0.1 µM in patients with overweight- obesity is likely to be due to cancer. Measurement of MG in patients with overweight/obesity is therefore justified.

### b. MG blood level in pre-cachectic or cachectic cancer patients

As indicated in Table 4, cancer patients with a BMI lower than 18 (i.e. with underweight or cachexia) have significant higher MG blood levels than patients with normal BMI (18<BMI<25). It was also found that MG blood levels are significantly inversely correlated with albumin blood levels (data not shown). Since hypo-albuminemia has been shown to be associated with cachexia, this confirms indirectly that in cancer patients, high MG blood levels are associated with cachexia. This result is displayed in Figure 7, in which, as previously indicated, MG blood levels in cancer patients are shown to be significantly inversely correlated with BMI (Fig 7B), whereas in normal subjects MG blood levels and BMI are not correlated (Fig 7A).

Since underweight-cachexia is associated with reduced survival time irrespective of tumor volume or presence of metastases, these data strongly suggest that repeated measurement of MG in cancer patients constitutes a new tool for predicting and early detecting cachexia and therefore for objectively assessing patient prognosis.

### Reference Example 4: Determination of a cachexia-related MG blood control value.

In cachexia the I/G index is increased or normal in 25 % of the cases respectively, and decreased in 50 % of the cases; depending on the advanced state of the cachexia, the lower the index, the lower severity of cachexia is. It is indeed well known that an increased I/G index relates to insulin resistance, while a decreased I/G index relates to deficient insulin secretion by β pancreatic cells. As displayed in Fig 8 in normal subjects the I/G index is constant whatever the value of MG blood levels is, whereas in cancer patients it is significantly inversely correlated with MG blood levels. On the basis of previous considerations (see above), the intersection point of the two curves define the limit from which the I/G index in cancer patients becomes lower than that in normal subjects. This intersection point therefore refers to a MG critical value - the so called "cachexia-related MG control value" - above which a lower insulin secretion occurs in cancer patients than in normal subjects, a finding that is clearly associated with cachexia. This means that the 0.2 µM MG blood control value that has been determined on the graph corresponds to the MG limit value above which cancer patients enter cachexia or severe pre-cachexia (Fig 8).

Measuring MG in the blood of cancer patients seems warranted in order to determine the level of insulin resistance in comparison with that of insulin pancreatic secretion, and to recognize objectively the entry of a cachectic or severe pre-cachectic state in these patients.

## Claims

1. An *in vitro* method for monitoring the therapeutic efficiency of any anti-cancer treatment administered to patients with lung, colorectal or pancreas cancer, comprising the steps of:
a) determining an initial pretreatment MG production level in a first blood, serum or plasma sample obtained from said patients,
b) determining a second MG production level in a second blood, serum or plasma sample obtained after treatment from said patients,
wherein said second sample is obtained at a given time after obtaining the first sample,
c) comparing said initial and said second MG production levels, wherein if said second MG production level is higher than said initial MG production level, said treatment is considered not to be efficient on said patients; whereas if said second MG production level is lower than said initial MG production level, said treatment is considered to be efficient on said patients.

2. The *in vitro* method of claims 1 wherein said sample is a blood sample.

3. The *in vitro* method of claims 1 wherein said sample is a serum sample.

4. The *in vitro* method of claims 1 wherein said sample is a plasma sample.

5. The *in vitro* method of claims 1 to 4 for monitoring the therapeutic efficiency of any anti-cancer treatment administered to patients with lung cancer.

6. The *in vitro* method of claims 1 to 4 for monitoring the therapeutic efficiency of any anti-cancer treatment administered to patients with colorectal cancer.

7. The *in vitro* method of claims 1 to 4 for monitoring the therapeutic efficiency of any anti-cancer treatment administered to patients with pancreas cancer.

## Patentansprüche

1. In-vitro-Verfahren zum Überwachen der therapeutischen Wirksamkeit einer Krebsbehandlung bei Patienten mit Lungen-, Kolorektal- oder Bauchspeicheldrüsenkrebs, umfassend die Schritte:
a) Feststellen einer anfänglichen MG-Produktionsmenge vor der Behandlung in einer ersten Blut-, Serum- oder Plasmaprobe, die von den Patienten erhalten wurde,
b) Feststellen einer zweiten MG-Produktionsmenge in einer zweiten Blut-, Serum- oder Plasmaprobe, die nach der Behandlung von den Patienten erhalten wurde,
wobei die zweite Probe zu einem bestimmten Zeitpunkt nach dem Erhalten der ersten Probe erhalten wird;
c) Vergleichen der anfänglichen und der zweiten MG-Produktionsmenge, wobei, wenn die zweite MG-Produktionsmenge höher als die anfängliche MG-Produktionsmenge ist, die Behandlung bei den Patienten als nicht wirksam betrachtet wird;
wobei, wenn die zweite MG-Produktionsmenge niedriger als die anfängliche MG-Produktionsmenge ist, die Behandlung bei den Patienten als wirksam betrachtet wird.

2. In-vitro-Verfahren nach Anspruch 1, wobei es sich bei der Probe um eine Blutprobe handelt.

3. In-vitro-Verfahren nach Anspruch 1, wobei es sich bei der Probe um eine Serumprobe handelt.

4. In-vitro-Verfahren nach Anspruch 1, wobei es sich bei der Probe um eine Plasmaprobe handelt.

5. In-vitro-Verfahren nach Anspruch 1 bis 4 zum Überwachen der therapeutischen Wirksamkeit einer Krebsbehandlung, die Patienten mit Lungenkrebs verabreicht wird.

6. In-vitro-Verfahren nach Anspruch 1 bis 4 zum Überwachen der therapeutischen Wirksamkeit einer Krebsbehandlung, die Patienten mit Kolorektalkrebs verabreicht wird.

7. In-vitro-Verfahren nach Anspruch 1 bis 4 zum Überwachen der therapeutischen Wirksamkeit einer Krebsbehandlung, die Patienten mit Bauchspeicheldrüsenkrebs verabreicht wird.

## Revendications

1. Une méthode *in vitro* pour contrôler l'efficacité thérapeutique de tout traitement anticancéreux administré à des patients atteints d'un cancer du poumon, colorectal ou du pancréas, comprenant les étapes consistant à :
a) déterminer le niveau initial de production de MG avant traitement dans un premier échantillon de sang, de sérum ou de plasma obtenu auprès desdits patients,
b) déterminer le deuxième niveau de production de MG dans un deuxième échantillon de sang, de sérum ou de plasma obtenu après traitement auprès desdits patients, dans laquelle ledit deuxième échantillon est obtenu à un moment donné après l'obtention du premier échantillon,
c) comparer ledit niveau initial et ledit deuxième niveau de production de MG, dans laquelle si ledit deuxième niveau de production de MG est supérieur au niveau de production initial de MG, le traitement est considérée comme non efficace sur lesdits patients; alors que si ledit second niveau de production de MG est inférieur audit niveau de production initial de MG, ledit traitement est considéré comme efficace sur lesdits patients.

2. La méthode *in vitro* selon la revendication 1, dans laquelle ledit échantillon est un échantillon de sang.

3. La méthode *in vitro* selon la revendication 1, dans laquelle ledit échantillon est un échantillon de sérum.

4. La méthode *in vitro* selon la revendication 1, dans laquelle ledit échantillon est un échantillon de plasma.

5. La méthode *in vitro* selon les revendications 1 à 4 pour surveiller l'efficacité thérapeutique de tout traitement anticancéreux administré aux patients atteints d'un cancer du poumon.

6. La méthode *in vitro* des revendications 1 à 4 pour surveiller l'efficacité thérapeutique de tout traitement anticancéreux administré aux patients atteints d'un cancer colorectal.

7. La méthode *in vitro* des revendications 1 à 4 pour surveiller l'efficacité thérapeutique de tout traitement anticancéreux administré aux patients atteints d'un cancer du pancréas.
